# DEMANDE DE BREVET EUROPEEN

(11) **EP 1 302 666 A1**
(43) Date de publication de la demande: **16.04.2003**
(21) Numéro de dépôt: 02292378.3
(22) Date de dépôt: 26.09.2002
(51) Int. Cl.: F04D 17/16, A61M 16/00

(54) **Turbine à deux étages notamment pour appareil d'assistance respiratoire**

(30) Priorité: 10.10.2001 FR 0113042
(71) Demandeur: TAEMA, F-92182 Antony Cédex (FR)
(72) Inventeur: Brunner, Delphine, 92160 Antony (FR); Aubatier, François, 78470 St. Remy les Chevreuse (FR)
(74) Mandataire: Pittis, Olivier

(57) **Abrégé**

L'invention porte sur un appareil d'assistance respiratoire équipé d'une turbine ou compresseur permettant de délivrer du gaz sous pression comprenant un carter (1) comportant un orifice d'entrée (2) de gaz, un moteur (6) comportant un axe-moteur (5) d'axe (AA), ledit axe-moteur (5) étant mobile en rotation autour de l'axe (AA) lorsqu'il est entraîné par ledit moteur (6), une première hélice (3) et une seconde hélice (4) montées solidairement sur ledit axe-moteur (5), une cloison interne (7), agencée dans ledit carter (1), intercalée entre les deux hélices (3, 4) de manière à former un premier étage (13) interne audit carter (1) contenant la première hélice (3) et un second étage (14) interne audit carter (1) contenant la seconde hélice (4), lesdits premier étage (13) et second étage (14) étant agencés en série, ladite cloison interne (7) comportant une ouverture (8) traversée par une partie de l'axe-moteur (5) et par laquelle le premier étage interne (13) est en communication fluidique avec le second étage interne (14), et un orifice de sortie (15) de gaz pour délivrer du gaz sous pression.

## Description

L'invention concerne un appareil d'assistance respiratoire ou de ventilation artificielle de patients équipé d'une turbine (ou compresseur) à deux étages permettant de générer un gaz à pression et débit non nuls, lequel gaz est subséquemment distribué aux voies aériennes supérieures d'une personne.

Dans les appareils d'assistance respiratoire ou de ventilation artificielle, il est usuel d'utiliser une ou plusieurs turbines ou compresseurs pour générer le gaz, à la pression et au débit souhaités, destinés à être administrés aux patients ; les termes "turbine" et "compresseur" étant totalement équivalents.

Les pressions maximum atteintes par ces types d'appareils sont habituellement de l'ordre de 50 à 60 hPa.

Dans certains appareils connus, deux turbines moyenne pression sont montées en série pour pouvoir atteindre la pression de gaz désirée . Dans ce cas, la sortie de la première turbine est reliée à l'entrée de la seconde turbine de sorte que les deux pressions s'additionnent en une pression résultante correspondant à la pression totale souhaitée.

L'avantage essentiel de cet arrangement est qu'il permet de réaliser une régulation de pression par simple variation des vitesses des turbines et ce, grâce à leur faible inertie.

Cependant, à l'inverse, les inconvénients qui en résultent sont que cette solution impose une parfaite synchronisation de la vitesse des deux turbines, une isolation acoustique lourde et coûteuse, un échauffement important et néfaste du gaz dû à son passage dans les deux turbines, et un fonctionnement sous oxygène pur n'est pas possible car les turbines ne le permettent pas.

Par ailleurs, il existe aussi des systèmes s'apparentant à des compresseurs basse pression. Un tel système comporte deux étages reliés en parallèle pour atteindre le débit souhaite. Tout l'ensemble est habituellement en alliage d'aluminium usiné, ce qui lui permet de fonctionner avec de l'oxygène pur.

Toutefois, si un tel système conduit à un faible encombrement, un faible bruit, à une consommation réduite et permet un fonctionnement avec un gaz formé de 100% O₂, il apparaît qu'il présente aussi les inconvénients d'être cher car tout est usiné et monté ensuite, et de présenter une inertie importante des pièces rotatives en métal, ce qui augmente considérablement le temps de réponse de l'appareil.

Le but de l'invention est alors de proposer un appareil de ventilation assistée de patient munie d'une turbine qui soit amélioré par rapport aux appareils à turbines ou compresseurs connus, c'est-à-dire un ventilateur à turbine pouvant générer à partir d'une alimentation électrique basse tension, classiquement de l'ordre de 6 volts à 24 volts, une pression gazeuse de 0 à 100 hPa ; pouvant utiliser avec un débit minimum de 120 I/mn à 100 hPa ; utiliser l'air ambiant ou enrichi en 0₂ (jusqu'à 100%), et étant d'encombrement et de bruit les plus réduits possible.

La solution de l'invention est alors un appareil d'assistance respiratoire ou de ventilation artificielle comportant une turbine, en particulier un appareil d'assistance respiratoire comportant un compartiment de mélange permettant de réaliser un mélange d'un premier gaz avec de l'oxygène et une turbine agencée en aval dudit compartiment de mélange de gaz, ladite turbine permettant de délivrer du gaz sous pression comprenant :
- un carter comportant un orifice d'entrée de gaz,
- un moteur comportant un axe-moteur d'axe (AA), ledit axe-moteur étant mobile en rotation autour de l'axe (AA) lorsqu'il est entraîné par ledit moteur,
- une première hélice et une seconde hélice montées solidairement sur ledit axe-moteur,
- une cloison interne, agencée dans ledit carter, intercalée entre les deux hélices de manière à former un premier étage interne audit carter contenant la première hélice et un second étage interne audit carter contenant la seconde hélice, lesdits premier étage et second étage étant agencés en série, ladite cloison interne comportant une ouverture traversée par une partie de l'axe-moteur et par laquelle le premier étage interne est en communication fluidique avec le second étage interne, et
- un orifice de sortie de gaz pour délivrer du gaz sous pression.

Selon le cas, l'appareil de l'invention peut comprendre l'une ou plusieurs des caractéristiques techniques suivantes :
- l'orifice d'entrée de gaz de la turbine communique avec le premier étage interne du carter, de préférence l'orifice d'entrée est d'axe (A-A).
- l'orifice de sortie de gaz de la turbine pour délivrer du gaz sous pression communique avec le second étage interne du carter.
- le moteur de la turbine est un moteur électrique, de préférence un moteur alimenté en courant électrique basse tension.
- la cloison interne de la turbine est solidaire de la paroi interne du carter, de préférence la cloison interne et le carter sont réalisés en plastique moulé par injection.
- les deux hélices de la turbine sont identiques ou différentes, de préférence les deux hélices sont munies d'aubes et sans flasque.
- le moteur est agencé à l'extérieur du carter.
- la paroi du second étage du carter de la turbine, située en regard du moteur, comprend un passage traversé par l'axe-moteur dudit moteur, de préférence des moyens d'étanchéité agencés au niveau dudit passage permettent d'éviter ou de minimiser toute sortie de gaz par ledit passage.
- il comporte une première ligne de gaz apte à véhiculer de l'air, ledit premier gaz jusque dans le compartiment de mélange de manière à alimenter le compartiment avec de l'air ledit premier gaz,
- il comporte une deuxième ligne de gaz apte à véhiculer de l'oxygène jusque dans le compartiment de mélange de manière à alimenter ledit compartiment de mélange avec de l'oxygène, la deuxième ligne de gaz comportant :
   - il comporte une vanne à ouverture proportionnelle permettant de contrôler le débit d'oxygène circulant dans ladite deuxième ligne de gaz, et
   - un capteur de débit d'oxygène permettant de mesurer le débit d'oxygène circulant dans ladite deuxième ligne de gaz, et
   - un capteur de débit mesurant le débit total du mélange gazeux.
   - la vanne à ouverture proportionnelle est une électrovanne.
   - la vanne à ouverture proportionnelle est commandée par des moyens de pilotage reliés à ladite vanne et/ou le capteur de débit d'oxygène est relié aux moyens de pilotage.

L'invention va maintenant être décrite plus en détail grâce à la description donnée ci-après en références aux figures annexées.

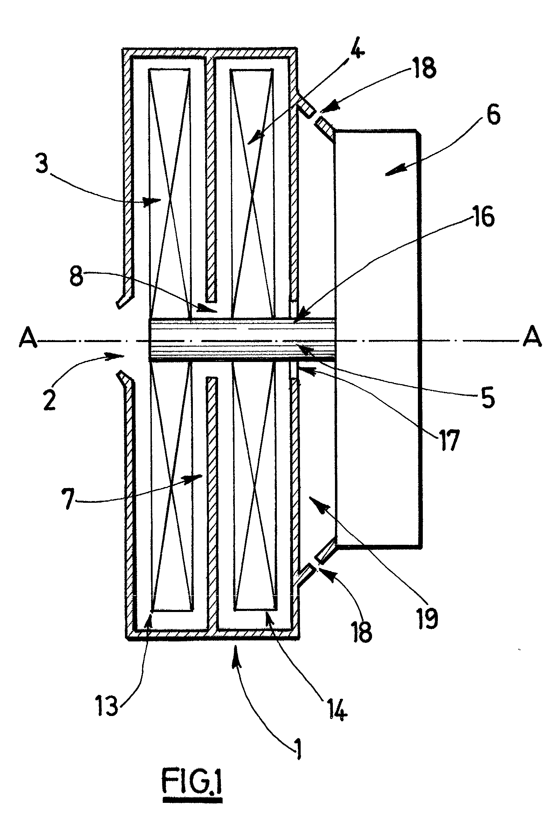

La figure 1 représente un schéma de l'architecture de principe (en coupe) d'une turbine équipant un appareil selon l'invention qui est un moto-générateur compact de débit et de pression destiné à être utilisé pour ventiler un patient avec de l'air ambiant ou enrichi en oxygène (jusqu'à 100%) et alimenté en énergie par un courant électrique basse tension, typiquement de 24 volts. Dans le cadre de l'invention, on utilise indifféremment et manière totalement équivalente, les termes turbine ou compresseur de gaz.

Elle peut délivrer une pression de 0 à 100 hPa avec un débit de 120 I/mn à la pression maximale. Les hélices 3,4 et le carter 1 sont réalisés dans un plastique chargé, moulé par injection, à haut point d'ignition compatible avec l'oxygène.

Cette turbine est reliée à une enceinte insonorisée ou carter 1 qui fait fonction de capacité d'entrée, le moteur 6 se trouvant à l'extérieur. Un apport d'oxygène en quantité adaptée à l'intérieur de ce volume permet de faire en amont de l'entrée 2 de la turbine, un mélange air/O₂ à la valeur de consigne que l'on insuffle au patient via la turbine.

La turbine est un système à deux étages 13, 14 en série fonctionnant par recyclage du gaz du premier étage 13 dans le second 14 par un dispositif adapté. Grâce à cette technologie, les performances maxi sont atteintes à une vitesse basse d'environ 15000 t/mn. Ceci permet une réduction du bruit et une durée de vie allongée .

Un système d'étanchéité 17 sur l'axe moteur 5 empêche toute sortie de gaz de l'ensemble hélices/carter (13, 14, 1) vers l'extérieur ou dans le moteur 6.

Une chambre de décompression 19 entre le moteur 6 et la turbine permet, via des orifices 18 d'évacuation adaptés, l'évacuation de l'oxygène en cas de défaillance du système d'étanchéité 17.

Le moteur 6 d'entraînement est du type sans balai, trois phases, 4 pôles à rotor extérieur avec trois capteurs effet Hall donnant le signal de rotation. Il est monté en bout d'axe -moteur 5.

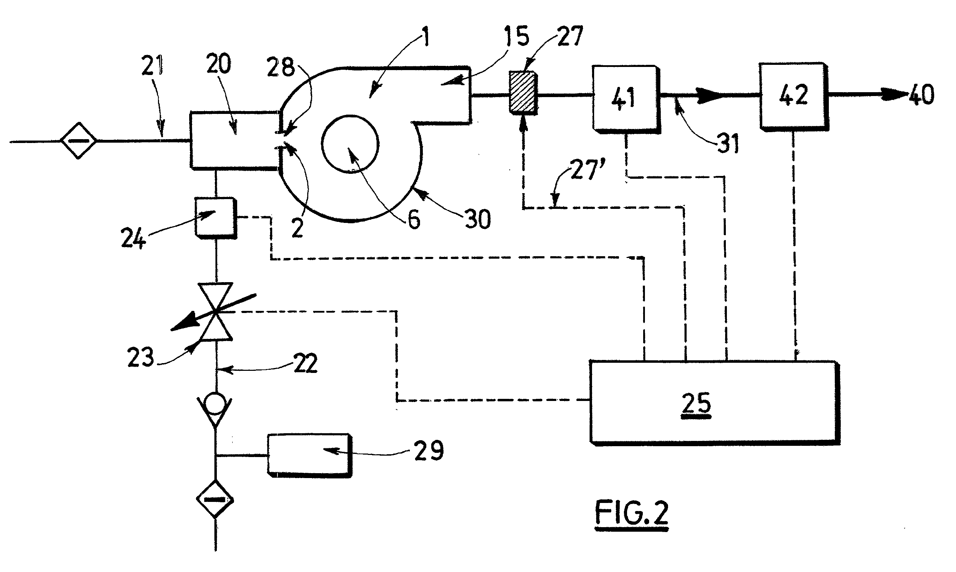

Sur la figure 2, on a schématisé un appareil d'assistance respiratoire équipé 30 conforme à l'invention d'une telle turbine, en amont de laquelle est agencé un compartiment de mélange 20 ou mélangeur faisant office de capacité de mélange et d'homogénéisation pour réaliser le mélange d'air avec de l'oxygène provenant par exemple d'une canalisation d'oxygène 21 d'un réseau hospitalier ou d'une bouteille de conditionnement d'oxygène.

L'air servant au mélange peut, quant à lui, soit provenir également d'une canalisation 22 d'air purifié d'un réseau hospitalier, soit être directement prélevé directement à l'atmosphère ; toutefois, dans ce dernier cas, il est recommandé de le soumettre préalablement à un traitement pour le purifier, par exemple une filtration des poussières et des microorganismes qui peuvent s'y trouver.

L'air est véhiculé par une première ligne 21 de gaz qui alimente le compartiment 20 de mélange, alors qu'une deuxième ligne 22 de gaz permet de véhiculer l'oxygène de manière à alimenter le compartiment 20 de mélange avec ce gaz de sorte d'y opérer le mélange des deux gaz dans les proportions désirées.

Ici, la deuxième ligne 22 de gaz comporte une vanne 23 à ouverture proportionnelle, préférentiellement une électrovanne, pouvant être alimentée directement par le réseau hospitalier ou par un réservoir, à des pressions situées entre environ 2,8 et 6 bars, permettant de contrôler le débit d'oxygène circulant dans la deuxième ligne 22 de gaz, un capteur de pression 29 et un capteur de débit 24 d'oxygène permettant de mesurer le débit d'oxygène circulant dans cette deuxième ligne 22.

La vanne 23 à ouverture proportionnelle est commandée par des moyens de pilotage 25 reliés à ladite vanne 23.

De manière analogue, le capteur de débit 24 d'oxygène est relié aux moyens de pilotage 25.

Pour assurer une détermination fiable du débit d'oxygène, le capteur de débit 24 d'oxygène est connecté à la deuxième ligne 22 de gaz entre la vanne 23 et le compartiment 20 de mélange.

En outre, une troisième ligne 28 de gaz, non détaillée, permet d'extraire du compartiment de mélange 20 au moins une partie du mélange gazeux d'air et d'oxygène en proportions désirées réalisé dans celui-ci et d'alimenter ensuite la turbine 30 selon l'invention, via son orifice d'entrée 2.

L'ensemble du système de l'invention est contrôlé et commandé par des moyens de pilotage 25 comprenant un calculateur ou un microprocesseur.

Pour permettre d'obtenir le mélange gazeux souhaité, par exemple contenant de 20% à environ 99% en volume d'oxygène, on contrôle le débit d'oxygène alimentant le compartiment de mélange 20 au moyen de la vanne proportionnelle 23 et du capteur de débit 24.

Par ailleurs, la ligne 31 de gaz comporte elle aussi un capteur de débit 27 d'air ou de mélange également relié aux moyens de pilotage 25.

Le mélange gazeux enrichi en oxygène dans le compartiment mélangeur 20, puis comprimé par la turbine 30 de l'invention est alors envoyé vers le patient 40 par l'intermédiaire d'un conduit-patient 31 relié à la sortie de la turbine 30, lequel conduit patient 31 comporte un capteur de débit mélange 27, une valve inspiratoire 41 et un capteur 42 de débit inspiratoire, tous trois reliés aux moyens de pilotage 25.

L'utilisation de pièces en plastique injecté et d'un moteur à trois phases conduisent à une turbine à bas prix, avec un gain de poids et de volume permettant de remplacer les turbines beaucoup plus bruyantes.

## Revendications

1. Appareil d'assistance respiratoire ou de ventilation artificielle comportant une turbine permettant de délivrer du gaz sous pression comprenant :
- un carter (1) comportant un orifice d'entrée (2) de gaz,
- un moteur (6) comportant un axe-moteur (5) d'axe (AA), ledit axe-moteur (5) étant mobile en rotation autour de l'axe (AA) lorsqu'il est entraîné par ledit moteur (6),
- une première hélice (3) et une seconde hélice (4) montées solidairement sur ledit axe-moteur (5),
- une cloison interne (7), agencée dans ledit carter (1), intercalée entre les deux hélices (3, 4) de manière à former un premier étage (13) interne audit carter (1) contenant la première hélice (3) et un second étage (14) interne audit carter (1) contenant la seconde hélice (4), lesdits premier étage (13) et second étage (14) étant agencés en série, ladite cloison interne (7) comportant une ouverture (8) traversée par une partie de l'axe-moteur (5) et par laquelle le premier étage interne (13) est en communication fluidique avec le second étage interne (14), et
- un orifice de sortie (15) de gaz pour délivrer du gaz sous pression.

2. Appareil selon la revendication 1, **caractérisée en ce que** l'orifice d'entrée (2) de gaz de la turbine communique avec le premier étage interne (13) du carter (1), de préférence l'orifice d'entrée (2) est d'axe (A-A).

3. Appareil selon l'une des revendications 1 ou 2, **caractérisée en ce que** l'orifice de sortie (15) de gaz de la turbine pour délivrer du gaz sous pression communique avec le second étage interne (14) du carter (1).

4. Appareil selon l'une des revendications 1 à 3, **caractérisée en ce que** le moteur (6) de la turbine est un moteur électrique, de préférence un moteur (6) alimenté en courant électrique basse tension.

5. Appareil selon l'une des revendications 1 à 4, **caractérisée en ce que** la cloison interne (7) de la turbine est solidaire de la paroi interne du carter (1), de préférence la cloison interne (7) et le carter (1) sont réalisés en plastique moulé par injection.

6. Appareil selon l'une des revendications 1 à 5, **caractérisée en ce que** les deux hélices (3, 4) de la turbine sont identiques ou différentes.

7. Appareil selon l'une des revendications 1 à 6, **caractérisée en ce que** le moteur (6) de la turbine est agencé à l'extérieur du carter (1).

8. Appareil selon l'une des revendications 1 à 7, **caractérisée en ce que** la paroi du second étage (14) du carter (1) de la turbine, située en regard du moteur (6), comprend un passage (16) traversé par l'axe-moteur (5) dudit moteur (6), de préférence des moyens d'étanchéité (17) agencés au niveau dudit passage (16) permettent d'éviter ou de minimiser toute sortie de gaz par ledit passage (16).

9. Appareil selon l'une des revendications 1 à 8, **caractérisée en ce que** les deux hélices (3, 4) de la turbine sont munies d'aubes et sans flasque.

10. Appareil d'assistance respiratoire selon l'une des revendications 1 à 9, comportant un compartiment de mélange (20) permettant de réaliser un mélange d'un premier gaz avec de l'oxygène, ladite turbine (30) étant agencée en aval dudit compartiment de mélange (20) de gaz.

11. Appareil selon la revendication 10, **caractérisé en ce qu'**il comporte, en outre :
- une première ligne (21) de gaz apte à véhiculer de l'air, ledit premier gaz jusque dans le compartiment (20) de mélange de manière à alimenter le compartiment (20) avec de l'air ledit premier gaz,
- une deuxième ligne (22) de gaz apte à véhiculer de l'oxygène jusque dans le compartiment (20) de mélange de manière à alimenter ledit compartiment (20) de mélange avec de l'oxygène, la deuxième ligne (22) de gaz comportant :
- une vanne (23) à ouverture proportionnelle permettant de contrôler le débit d'oxygène circulant dans ladite deuxième ligne (22) de gaz, et
- un capteur de débit (24) d'oxygène permettant de mesurer le débit d'oxygène circulant dans ladite deuxième ligne (22) de gaz, et
- un capteur de débit (27) mesurant le débit total du mélange gazeux.

12. Appareil selon la revendication 11, **caractérisé en ce que** la vanne (23) à ouverture proportionnelle est une électrovanne et/ou la vanne (23) à ouverture proportionnelle est commandée par des moyens de pilotage (25) reliés (23') à ladite vanne (23) et/ou le capteur de débit (24) d'oxygène est relié (24') aux moyens de pilotage (25).
